## Europäisches Patentamt
## European Patent Office
## Office européen des brevets

(19)

(11) Veröffentlichungsnummer: **0 141 928**
**B1**

(12) **EUROPÄISCHE PATENTSCHRIFT**

(45) Veröffentlichungstag der Patentschrift:
14.01.87

(21) Anmeldenummer: **84109627.4**

(22) Anmeldetag: **13.08.84**

(51) Int. Cl.⁴: **C 07 C 143/24**, C 07 C 139/00

(54) **Verfahren zur Sulfonierung von aromatischen Verbindungen mit Schwefeltrioxid.**

(30) Priorität: **23.08.83 · DE 3330334**

(43) Veröffentlichungstag der Anmeldung:
**22.05.85 Patentblatt 85/21**

(45) Bekanntmachung des Hinweises auf die Patenterteilung:
**14.01.87 Patentblatt 87/3**

(84) Benannte Vertragsstaaten:
**CH DE FR GB LI**

(56) Entgegenhaltungen:
**DE-A-1 493 060**
**DE-A-2 353 918**
**DE-A-2 728 070**
**US-A-3 155 716**

(73) Patentinhaber: **BAYER AG, Konzernverwaltung RP Patentabteilung, D-5090 Leverkusen 1 Bayerwerk (DE)**

(72) Erfinder: **Behre, Horst, Dr., Zur alten Linde 12, D-5068 Odenthal (DE)**
Erfinder: **Blank, Heinz Ulrich, Dr., Am Geusfelde 33, D-5068 Odenthal (DE)**
Erfinder: **Köhler, Wilfried, Dr., Gustav- Freitag-Strasse 2, D-5090 Leverkusen (DE)**
Erfinder: **Müller, Nikolaus, Dr., Walter- Flex-Strasse 32, D-5090 Leverkusen (DE)**
Erfinder: **Schnegg, Peter, Dr., Heidberger Strasse 44, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

## Beschreibung

Die Erfindung betrifft ein neues Verfahren zur Herstellung aromatischer Sulfonsäuren durch Sulfonierung aromatischer Verbindungen mit Schwefeltrioxid.

Die Herstellung aromatischer Sulfonsäuren durch Sulfonierung aromatischer Verbindungen mit Schwefeltrioxid ist bekannt (s. z.B. E. E. Gilbert, Chemical Revue 62 (1962) Seiten 549 - 589). Nachteilig an der Verwendung von Schwefeltrioxid als Sulfonierungsmittel ist, daß bei diesen Sulfonierungen wegen der hohen Reaktionsfähigkeit des Schwefeltrioxids erhebliche Mengen an unerwünschten, oftmals schwer abtrennbaren Nebenprodukten entstehen.

Eine wesentlich schonsamere Sulfonierung mit Schwefeltrioxid wird erreicht, wenn man die Reaktionsfähigkeit des Schwefeltrioxids durch Komplexbildung mit Äthern wie Dioxan, tert.-Aminen wie Pyridin oder Carbonsäureamiden wie Dimethylformamid herabsetzt. Die Sulfonierungen mit diesen Schwefeltrioxid-Komplexen verlaufen wesentlich selektiver, d.h. unter Bildung weniger Nebenprodukte, haben jedoch den vor allem für Sulfonierungen im technischen·Maßstab schwerwiegenden Nachteil, daß die in stöchiometrischen Mengen anfallenden Komplexierungsmittel aus Kostengründen und aus Gründen der Abwasserbelastung zurückgewonnen werden müssen, bzw. wenn sie nicht zurückgewonnen werden, zu einer starken Belastung des Abwassers führen und damit den Vorteil, den die Sulfonierung mit Schwefeltrioxid an sich bietet, zumindest zum Teil wieder zunichte machen.

An Stelle des reaktionsfähigen Schwefeltrioxids hat man auch bereits Chlorsulfonsäure als Sulfonierungsmittel verwendet. Infolge seiner verglichen mit Schwefeltrioxid schwächeren Sulfonierungswirkung wird mit Chlorsulfonsäure, insbesondere bei der Sulfonierung leicht sulfonierbarer aromatischer Verbindungen, eine verbesserte Selektivität und damit eine Herabsetzung der Nebenprodukt-Bildung erreicht. Gemäß einer Angabe in der US-A-3 155 716 soll die für die Sulfonierung zu verwendende Chlorsulfonsäure in situ durch Reaktion von Schwefeltrioxid mit Chlorwasserstoff herstellbar sein. Die Verwendung von Chlorsulfonsäure als Sulfonierungsmittel hat jedoch, unabhängig davon, ob sie als solche eingesetzt oder in situ erzeugt wurde, den schwerwiegenden Nachteil, dan als Nebenprodukt stöchiometrische Mengen an Chlorwasserstoff anfallen, die vernichtet, d.h. durch Neutralisation unschädlich gemacht werden müssen, da ihre Wiederverwendung zur Herstellung von Chlorsulfonsäure wegen des damit verbundenen hohen technischen Aufwandes nicht in Betracht kommt. D.h. obwohl Chlorsulfonsäure an sich ein günstiges Sulfonierungsmittel für empfindliche aromatische Verbindungen ist, steht ihrer Verwendung für Sulfonierungen in technischem Maßstab entgegen:

a) ihr verglichen mit dem Schwefeltrioxid-Preis erheblich höherer Preis und

b) die hohen Kosten, die mit dem Unschädlichmachen des bei der Sulfonierung in äquimolaren Mengen entstehenden Chlorwasserstoffs verbunden sind.

Überraschenderweise wurde nun gefunden, daß Halogenwasserstoff ein ausgezeichnetes Agenz ist, die Reaktionsfähigkeit von Schwefeltrioxid herabzusetzen. Es wurde gefunden, daß die Reaktionsfähigkeit von Schwefeltrioxid durch Halogenwasserstoff soweit herabgesetzt wird, daß es nunmehr gelingt auch empfindliche aromatische Verbindungen, die wegen ihrer leichten Sulfonierbarkeit eine schonsame Sulfonierung erfordern, in hervorragenden Ausbeuten selektiv mit Schwefeltrioxid zu sulfonieren. Es wurde gefunden, daß man die Reaktionsfähigkeit des Schwefeltrioxids durch Einstellen einer gewissen Halogenwasserstoffkonzentration im Reaktionsgeaisch der Sulfonierbarkeit der zu sulfonierenden aromatischen Verbindungen anpassen kann.

Die Erfindung betrifft daher ein Verfahren zur selektiven Monosulfonierung aromatischer Verbindungen mit Schwefeltrioxid in inerten organischen Lösungsmitteln, das dadurch gekennzeichnet ist daß man die Sulfonierung in Gegenwart von unterstöchiometrischen Mengen (bezogen auf Schwefeltrioxid) Halogenwasserstoff vornimmt.

Als Halogenwasserstoff wird vorzugsweise Chlorwasserstoff verwendet. Der Halogenwasserstoff kann dem Sulfonierungsgemisch als solcher, z.B. als gasförmiger Chlorwasserstoff, oder aber in Form von Verbindungen zugesetzt werden, die im Reaktionsgemisch unter den Reaktionsbedingungen Halogenwasserstoff abspalten oder bilden. Eine solche im Reaktionsgemisch unter den Reaktionsbedingungen Halogenwasserstoff (Chlorwasserstoff) bildende Verbindung ist z.B. Chlorsulfonsäure.

Der Halogenwasserstoff kann der zu sulfonierenden Verbindung vor der Zugabe des Schwefeltrioxids oder aber gleichzeitig mit dem Schwefeltrioxid zugesetzt werden.

Die desaktivierende Wirkung des Halogenwasserstoffs ist abhängig von der Menge, in der er eingesetzt wird; je größer die Halogenwasserstoffmenge, um so stärker die Desaktivierung des Schwefeltrioxids. Infolgedessen werden bei der Sulfonierung besonders empfindlicher, leicht zu sulfonierender aromatischer Verbindungen größere Mengen an Halogenwasserstoff eingesetzt als bei der Sulfonierung weniger empfindlicher und weniger leicht zu sulfonierender aromatischer Verbindungen. Im allgemeinen reicht es zur gewünschten Desaktivierung des Schwefeltrioxids aus, wenn man den Halogenwasserstoff in einer Menge von 0,01 - 0,9 Mol, vorzugsweise 0,1 - 0,8 Mol, insbesondere 0,25 - 0,75 Mol Halogenwasserstoff je Mol Schwefeltrioxid einsetzt.

Das erfindungsgemäße Verfahren eignet sich besonders für die selektive Monosulfonierung leicht zu sulfonierender aromatischer Verbindungen wie Naphthalin, 1-Methylnaphthalin, 2-Hydroxynaphthalin und Diphenyl. Diese Verbindungen werden mit Hilfe des erfindungsgemäßen Verfahrens in hohen Ausbeuten selektiv zu Naphthalin-1-sulfonsäure, 1-Methylnaphthalin-4-sulfonsäure, 2-Hydroxynaphthalin-1-sulfonsäure bzw. Diphenyl-4-sulfonsäure sulfoniert.

Während bei der Aufarbeitung der Sulfonierungsgemische, die bei der Sulfonierung aromatischer Verbindungen mit Chlorsulfonsäure anfallen, die bei der Reaktion gebildeten äquimolaren Mengen an Chlorwasserstoff vernichtet oder aber mittels aufwendiger Verfahren in weiterverwendbaren reinen Chlorwasserstoff oder reine Salzsäure oder in Chlorsulfonsäure überführt werden müssen, werden die beim erfindungsgemäßen Verfahren anfallenden Halogenwasserstoff-haltigen Sulfonierungsgemische in der Weise aufarbeitet, daß der Halogenwasserstoff nicht verloren geht, sondern z.B. zusammen mit dem organischen Lösungsmittel zurückgewonnen und im nächsten Ansatz wiederverwendet wird.

Das erfindungsgemäße Verfahren ist deshalb von großer technischer Bedeutung, weil es die selektive Sulfonierung empfindlicher aromatischer Verbindungen mit Schwefeltrioxid ermöglicht, ohne daß bei der Sulfonierung Fremdverbindungen wie Komplexbildner oder Chlorwasserstoff freigesetzt bzw. gebildet werden, die entweder zurückgewonnen oder aber unschädlich gemacht werden müssen. Beim erfindungsgemäßen Verfahren entstehen außer den gewünschten aromatischen Sulfonsäuren und vernachlässigbar geringen Mengen an Nebenprodukten keinerlei Fremdverbindungen. Der Halogenwasserstoff wird zurückgewonnen und im nächsten Ansatz wiederverwendet.

Als inerte organische Lösungsmittel eignen sich für das erfindungsgemäße Verfahren solche Lösungsmittel, die mit Schwefeltrioxid unter den Reaktionsbedingungen nicht oder zumindest in nicht nennenswertem Maße reagieren und die zugleich ein gutes Lösungsvermögen für den Halogenwasserstoff aufweisen. Solche Lösungsmittel sind z.B. aliphatische Halogenkohlenwasserstoffe wie Tetrachlorethan, 1,2-Dichlorethan und 1,2-Dichlorpropan. Besonders bewährt hat sich Dichlormethan.

Das erfindungsgemäße Verfahren wird bei Temperaturen von -40 bis +20°C, vorzugsweise -30 bis +10°C, insbesondere -20 bis 0°C durchgeführt.

Das Schwefeltrioxid kann im erfindungsgemäßen Verfahren in flüssiger oder gasförmiger Form oder in Form einer Lösung im inerten organischen Lösungsmittel eingesetzt werden. Vorzugsweise werden gasförmiges Schwefeltrioxid, gegebenenfalls verdünnt mit einem Inertgas wie Stickstoff, oder Schwefeltrioxidlösungen in Dichlormethan verwendet.

Das erfindungsgemäße Sulfonierungsverfahren kann auf verschiedene Weise durchgeführt werden; z.B. in der Weise, da- man die zu sulfonierende aromatische Verbindung, z.B. Naphthalin, in dem inerten organischen Lösungsmittel, z.B. Dichlormethan, löst oder suspendiert und solange Halogenwasserstoff, z.B. Chlorwasserstoff, in die Lösung oder Suspension einleitet, bis die Lösung oder Suspension die gewünschte Mengen an Halogenwasserstoff aufgenommen hat. Anschließend wird das Schwefeltrioxid zugegeben. Die Sulfonierung kann sowohl unter Normaldruck als auch unter erhöhtem Druck durchgeführt werden. Durch geeignete Wahl der Parameter Druck und Temperatur sowie eines inerten organischen Lösungsmittels mit dem gewünschten Lösungsvermögen für den Halogenwasserstoff, lassen sich verschiedene stationäre Halogenwasserstoff-Konzentrationen im Reaktionsgemisch während der Schwefeltrioxid-Zugabe einstellen.

Eine andere Weise der Ausführung besteht darin, einer Lösung oder Suspension der zu sulfonierenden aromatischen Verbindung in dem organischen Lösungsmittel gleichzeitig den Halogenwasserstoff und das Schwefeltrioxid zuzusetzen.

Eine dritte Weise besteht darin, den für das erfindungsgemäße Verfahren erforderlichen Halogenwasserstoff unmittelbar im Reaktionsgemisch zu erzeugen, z.B. indem man die aromatische Verbindung in dem inerten organischen Lösungsmittel zunächst partiell mit Chlorsulfonsäure sulfoniert, wobei man dafür Sorge trägt, daß der gebildete Chlorwasserstoff in der Reaktionsmischung verbleibt und anschließend die Sulfonierung durch Zugabe der für die vollständige Sulfonierung erforderlichen Menge an Schwefeltrioxid zu Ende führt. Anstatt die gesamte Chlorsulfonsäure zu Beginn zuzusetzen, kann man bei dieser Ausführungsform zunächst auch nur einen Teil der Chlorsulfonsäure zusetzen und nachdem sich dieser unter Bildung von Chlorwasserstoff umgesetzt hat, den verbleibenden Teil Chlorsulfonsäure gleichzeitig mit der für die Sulfonierung erforderlichen Menge an Schwefeltrioxid zudosieren.

Die nach dem erfindungsgemäßen Verfahren erhältlichen Sulfonsäuren wie Naphthalin-1-sulfonsäure, 2-Hydroy-naphthalin-1-sulfonsäure und Diphenyl-4-sulfonsäure sind wichtige Vorprodukte und Zwischenprodukte für die Herstellung von Farbstoffen, Pflanzenschutzmitteln und Emulgatoren (s. z.B. Ullmanns Enzyklopädie der technischen Chemie 4. Auflage Bd. 17, Seiten 117 und 94 und Bd. 18, Seite 219).

**Beispiel 1**

In einer 1-Liter-Sulfier-Apparatur werden 64,1 g (0,5 Mol) Naphthalin in 250 ml wasserfreiem Dichlormethan gelöst. Die Lösung wird auf -20° C abgekühlt. In die entstehende Suspension leitet man unter Rühren zunächst innerhalb von etwa 30 Minuten 9,2 g (0,25 Mol) Chlorwasserstoff-Gas ein. Anschließend werden ebenfalls unter Rühren bei -20° C innerhalb 1 Stunde 40 g (0,5 Mol) gasförmiges Schwefeltrioxid mittels trockenem Stickstoff auf die Oberfläche der gerührten Naphthalin/Dichlormethan-Suspension geleitet. Die Reaktionsmischung wird anschließend 2 Stunden bei -20° C gerührt und dann in 500 g Eis-Wasser-Gemisch eingetragen.

Die Dichlormethan-Phase wird abgetrennt, 2 x mit je 250 ml Wasser extrahiert und im Vakuum zur Trockne eingeengt. Der Rückstand (5,9 g) enthält gemäß gaschromatographischer Analyse 73,2 Gew.-% Naphthalin ( = 6,7 % des eingesetzten Naphthalins).

Die vereinigten wäßrigen Phasen werden zum Entfernen von Dichlormethan-Resten im Vakuum kurz andestilliert, in einen 1-Liter-Meßkolben überführt und auf 1 Liter aufgefüllt.

Die Hochdruckflüssigkeitschromatographie (HPLC) dieser Lösung ergab folgenden Gehalt an Naphthalinsulfonsäuren:. 89,0 g Naphthalin-1-sulfonsäure ( = 85 % der Theorie bezogen auf eingesetztes Naphthalin; = 91,8 % der der Theorie bezogen auf umgesetztes Naphthalin)

6,2 g Naphthalin-2-sulfonsäure ( = 6,0 % der Theorie bezogen auf eingesetztes Naphthalin; = 6,5 % der Theorie bezogen auf umgesetztes Naphthalin)

0,4 g Naphthalin-disulfonsäuren ( = 0,28 % der Theorie bezogen auf eingesetztes Naphthalin; = 0,3 % der Theorie bezogen auf umgesetztes Naphthalin)

Die Sulfonierung wurde wie vorstehend beschrieben wiederholt, mit dem einzigen Unterschied, daß kein Chlorwasserstoff eingeleitet wurde. Die Ausbeuten an Naphthalin-sulfonsäuren (in % der Theorie bezogen auf umgesetztes Naphthalin) betrugen in diesem Fall:

76,0 % Naphthalin-1-sulfonsäure
8,4 % Naphthalin-2-sulfonsäure
0,4 % Naphthalin-1,3-disulfonsäure
10,4 % Naphthalin-1,5-disulfonsäure
2,7 % Naphthalin-1,6-disulfonsäure
0,6 % Naphthalin-1,7-disulfonsäure

**Beispiel 2**

Es wurde wie in Beispiel 1 beschrieben verfahren, nur wurden Chlorwasserstoff und Schwefeltrioxid gleichzeitig zudosiert.

Die Ausbeuten an Naphthalin-sulfonsäuren (in % der Theorie bezogen auf umgesetztes Naphthalin) betrugen:

90,4 % Naphthalin-1-sulfonsäure
7,3 % Naphthalin-2-sulfonsäure
0,3 % Naphthalin-disulfonsäuren

**Beispiel 3**

Es wurde wie in Beispiel 1 beschrieben verfahren, nur wurde in verdünnterer Lösung (die 64,1 g Naphthalin wurden in 500 ml Dichlormethan gelöst) gearbeitet.

Bei dieser Arbeitsweise betrugen die Ausbeuten an Naphthalin-sulfonsäuren (in % der Theorie bezogen auf umgesetztes Naphthalin):

89,9 % Naphthalin-1-sulfonsäure
9,8 % Naphthalin-2-sulfonsäure
0,5 % Naphthalin-disulfonsäuren

**Beispiel 4**

In der in Beispiel 1 beschriebenen Sulfier-Apparatur wird eine Lösung von 25,6 g (0,2 Mol) Naphthalin in 250 ml trockenem Dichlormethan auf -20° C abgekühlt. Durch die Lösung wird trockener Chlorwasserstoff mit einer Geschwindigkeit von 8 Liter/h geleitet und gleichzeitig innerhalb von 2 Stunden eine auf -10° C abgekühlte Lösung von 16 g (0,2 Mol) Schwefeltrioxid in 150 ml trockenem Dichlormethan bei -20° C unter Kühlen und Rühren zugesetzt.

Die Reaktionsmischung wird 2 Stunden bei -20° C nachgerührt und anschließend wie in Beispiel 1 beschrieben aufgearbeitet.

Gemäß HPLC beträgt die Ausbeute an Naphthalin-Sulfonsäuren (in % der Theorie bezogen auf umgesetztes Naphthalin):

89,0 % Naphthalin-1-sulfonsäure
10,0 % Naphthalin-2-sulfonsäure
0,6 % Naphthalin-1,5-disulfonsäure
0,2 % Naphthalin-1,6-disulfonsäure

**Beispiel 5**

In der in Beispiel 1 beschriebenen Sulfier-Apparatur wird die Lösung von 25,6 g (0,2 Mol) Naphthalin in 250 g trockenen Dichlormethan auf -20° C abgekühlt. Bei -20° C läßt man unter Rühren zunächst eine Lösung von 18 g (0,15 Mol) Chlorsulfonsäure in 50 g trockenem Dichlormethan innerhalb von 10 Minuten und anschließend eine auf -10° C gekühlte Lösung von 4 g (0,05 Mol) Schwefeltrioxid in 50 g trockenem Dichlormethan innerhalb von 20 Minuten einlaufen. Die Reaktionsmischung wird 2 Stunden bei -20° C nachgerührt und anschließend wie in Beispiel 4 beschrieben aufgearbeitet.

Gemäß HPLC beträgt die Ausbeute an Naphthalin-sulfonsäuren (in % der Theorie bezogen auf umgesetztes Naphthalin):

91,0 % Naphthalin-1-sulfonsäure
8,6 % Naphthalin-2-sulfonsäure

0,1 % Naphthalin-1,5-disulfonsäure
0,1 % Naphthalin-1,6-disulfonsäure
Bei gleichzeitiger Zugabe von Chlorsulfonsäure- und Schwefeltrioxid-Lösung betrug die Ausbeute an Naphthalin-Sulfonsäuren (in % der Theorie bezogen auf umgesetztes Naphthalin):
89,7 % Naphthalin-1-sulfonsäure
9,2 % Naphthalin-2-sulfonsäure
0,7 % Naphthalin-1,5-disulfonsäure
0,3 % Naphthalin-1,6-disulfonsäure

**Beispiel 6**

Es wurde wie in Beispiel 5 beschrieben gearbeitet, nur wurden anstelle der in Beispiel 5 verwendeten Lösungen von Chlorsulfonsäure und Schwefeltrioxid die Lösungen von 12 g (0,1 Mol) Chlorsulfonsäure und 8 g (0,1 Mol) Schwefeltrioxid jeweils in 50 g Dichlormethan innerhalb von 30 Minuten bzw. 90 Minuten eingetropft.
Gemäß HPLC betrug die Ausbeute an Naphthalin-sulfonsäuren (in % der Theorie bezogen auf umgesetztes Naphthalin):
89,0 % Naphthalin-1-sulfonsäure
10,3 % Naphthalin-2-sulfonsäure
0,3 % Naphthalin-1,5-disulfonsäure
0,2 % Naphthalin-1,6-disulfonsäure

**Bespiel 7**

In einer 2-Liter-Sulfonier-Apparatur werden 154 g (1.00 Mol) Diphenyl in 1000 ml wasserfreiem Dichlormethan gelöst. In die Lösung leitet man unter Rühren bei -10° C 7,3 g (0.20 Mol) Chlorwasserstoff-Gas ein. Anschließend werden ebenfalls unter Rühren bei -10° C innerhalb 2 h 77 g (0.96 Mol) gasförmiges Schwefeldioxid auf die Oberfläche der Lösung von Diphenyl und Chlorwasserstoff in Dichlormethan geleitet. Die Reaktionsmischung wird 1 h bei -10oC nachgerührt. Das Sulfoniergemisch wird wie in Beispiel 1 beschrieben aufgearbeitet.
Gemäß HPLC beträgt die Ausbeute an Diphenyl-sulfonsäuren (in % der Theorie bezogen auf umgesetztes Diphenyl):
99.4 % Diphenyl-4-sulfonsäure
0.6 % Diphenyl-4,4'-disulfonsäure.

**Beispiel 8**

Die Lösung von 30,8 g (0,2 Mol) Diphenyl in 250 g trockenem Dichlormethan wird, wie in Beispiel 4 beschrieben, unter Durchleiten von ca. 8 Liter/h trockenem Chlorwasserstoff mit einer auf -10° C gekühlten Lösung von 16 g (0,2 Mol) Schwefeltrioxid in trockenem Dichlormethan umgesetzt. Das Sulfonierungsgemisch wird wie in Beispiel 4 beschrieben aufgearbeitet.
Gemäß HPLC beträgt die Ausbeute an Diphenyl-sulfonsäuren (in % der Theorie bezogen auf umgesetztes Diphenyl):
98,3 % Diphenyl-4-sulfonsäure
0,7 % Diphenyl-4,4'-disulfonsäure.

**Beispiel 9**

In der in Beispiel 1 beschriebenen Sulfier-Apparatur werden 28,8 g (0,2 Mol) 2-Hydroxynaphthalin in 300 g trockenem Dichlormethan bei 40° C gelöst. Die Lösung wird auf -20° C abgekühlt. Durch die entstandene Suspension wird gleichmäßig trockener Chlorwasserstoff mit einer Geschwindigkeit von 8 Liter/h geleitet und gleichzeitig im Verlauf von etwa 1 Stunde eine auf -10° C abgekühlte Lösung von 16 g (0,2 Mol) Schwefeltrioxid in 50 g trockenem Dichlormethan bei -20° C unter Kühlen und Rühren zugesetzt. Anschließend wird die Reaktionsmischung 2 Stunden bei -20° C nachgerührt und dann unter Einhaltung einer Temperatur unter 20° C in etwa 100 g Eis-Wasser-Gemisch eingetragen. In der auf diese Weise erhaltenen 2-phasigen Reaktionsmischung wird durch Zugabe von 50 %iger Natronlauge ein pH-Wert von 7 eingestellt. Nach dem Abtrennen der Dichlormethan-Phase wird die wäßrige Phase durch Andestillieren im Vakuum von Lösungsmittel-Resten befreit, in einen Meßkolben überführt und mit Wasser auf 1 Liter aufgefüllt.
Gemäß HPLC betragen die Ausbeuten an 2-Hydroxynaphthalinsulfonsäuren (in % der Theorie bezogen auf umgesetztes 2-Hydroxynaphthalin):
92,8 % 2-Hydroxynaphthalin-1-sulfonsäure
0,5 % 2-Hydroxynaphthalin-5-sulfonsäure
0,5 % 2-Hydroxynaphthalin-6-sulfonsäure
3,0 % 2-Hydroxynaphthalin-8-sulfonsäure
0,6 % 2-Hydroxynaphthalin-1,6-disulfonsäure
Weitere 2-Hydroxynaphthalin-sulfonsäuren waren im Reaktionsgemisch nicht nachweisbar.
Wurde die vorstehend beschriebene Sulfonierung von 2-Hy-droxynaphthalin in Abwesenheit von Chlorwasserstoff vorgenommen, so wurden folgende Ausbeuten an 2-Hydroxynaph-thalin-sulfonsäuren (in % der Theorie bezogen auf umgesetztes 2-Hydroxynaphthalin) erhalten:
83,5 % 2-Hydroxynaphthalin-1-sulfonsäure
2,1 % 2-Hydroxynaphthalin-5-sulfonsäure
0,4 % 2-Hydroxynaphthalin-6-sulfonsäure
6,9 % 2-Hydroxynaphthalin-8-sulfonsäure
1,0 % 2-Hydroxynaphthalin-1,6-disulfonsäure
0,4 % 2-Hydroxynaphthalin-6,8-disulfonsäure

**Beispiel 10**

In einem 1-Liter-Sulfierbecher, der mit einem nach oben mit einer Glasfritte abschließenden Bodenauslauf versehen ist, wird eine Lösung von 64,1 g (0,5 Mol) Naphthalin in 250 ml wasserfreiem Dichlormethan auf -20°C abgekühlt. In die entstandene Suspension wird bei -20°C trockener Chlorwasserstoff bis zur Sättigung (das ist bis zur Aufnahme von etwa 15 g HCl) eingeleitet. Anschließend werden innerhalb von etwa 1,5 Stunden 40 g (0,5 Mol) Schwefeltrioxid bei -20°C unter Rühren auf die Oberfläche der Suspension von Naphthalin in chlorwasserstoffhaltigen Dichlormethan geleitet. Nach 2-stündigem Nachrühren der Reaktionsmischung bei -20°C wird der Niederschlag von der chlorwasserstoffhaltigen Mutterlauge isoliert, indem man diese Mutterlauge mittels trockenem Stickstoff durch die Fritte und den Bodenauslauf in einen zweiten gleichartig ausgerüsteten 1 Liter-Sulfierbecher drückt und den auf der Fritte befindlichen Niederschlag mit 50 ml trockenen Dichlormethan nachwäscht.

Die Zusammensetzung des abgesaugten Dichlormethan-feuchten Produktes wurde durch HPLC bestimmt; sie betrug:.

91,6 Gew.-% Naphthalin-1-sulfonsäure
2,3 Gew.-% Naphthalin-2-sulfonsäure
0,6 Gew.-% Naphthalin-disulfonsäuren

Die im zweiten Sulfierbecher vereinigte Lösung aus Filtrat und Waschlösung wird auf -20°C abgekühlt und mit 64,1 g (0,5 Mol) feingepulvertem Naphthalin versetzt.

In die Suspension wird wieder trockener Chlorwasserstoff bis zur Sättigung (es sind hierfür etwa 4 g HCl erforderlich) eingeleitet. Anschließend wird die Sulfonierung wie im vorhergehenden Ansatz durch Aufleiten von Schwefeltrioxid auf die Oberfläche der gerührten Suspension von Naphthalin in chlorwasserstoffhaltigem Dichlormethan durchgeführt. Nach beendeter Umsetzung wird die Mutterlauge wieder mittels trockenem Stickstoff in den ersten Sulfierbecher zurückgedrückt und das auf der Fritte befindliche Produkt mit frischem trockenen Dichlormethan gewaschen.

Die Zusammensetzung des Dichlormethan-feuchten Produktes wurde mittels HPLC bestimmt; sie betrug:

86,4 Gew.-% Naphthalin-1-sulfonsäure
4,1 Gew.-% Naphthalin-2-sulfonsäure
0,3 Gew.-% Naphthalin-disulfonsäuren

Die Ausbeuten an Sulfonsäuren (in % der Theorie bezogen auf umgesetztes Naphthalin) betragen bei dieser halbkontinuierlichen Arbeitsweise nach Einstellung der Sättigungskonzentration für die einzelnen Sulfonsäuren in der Mutterlauge, d.h. nach dem 5. Ansatz, je Ansatz:

91,7 % Naphthalin-1-sulfonsäure
6,2 % Naphthalin-2-sulfonsäure
0,3 % Naphthalin-disulfonsäuren

## Patentansprüche

1. Verfahren zur selektiven Monosulfonierung aromatischer Verbindungen mit Schwefeltrioxid in organischen Lösungsmitteln, dadurch gekennzeichnet, daß man die Sulfonierung in Gegenwart von 0,01 bis 0,9 Mol Halogenwasserstoff je Mol Schwefeltrioxid vornimmt.

2. Verfahren nach Anspruch 1, dadurch gekennzeichnet, daß als Halogenwasserstoff Chlorwasserstoff verwendet wird.

3. Verfahren nach Ansprüchen 1 und 2, dadurch gekennzeichnet, daß man die Sulfonierung bei Temperaturen von -40°C bis +20°C durchführt.

4. Verfahren nach Ansprüchen 1 bis 3, dadurch gekennzeichnet, daß man als organisches Lösungsmittel Dichlormethan verwendet.

## Claims

1. Process for the selective monosulphonation of aromatic compounds with sulphur trioxide in organic solvents, characterised in that the sulphonation is carried out in the presence of 0.01 to 0.9 mol of hydrogen halide per mol of sulphur trioxide.

2. Process according to Claim 1, characterised in that hydrogen chloride is used as the hydrogen halide.

3. . Process according to Claims 1 and 2, characterised in that the sulphonation is carried out at temperatures of -40°C to +20°C

4. Process according to Claims 1 to 3, characterised in that dichloromethane is used as the organic solvent.

## Revendications

1. Procédé de monosulfonation sélective de composés aromatiques à l'anhydride sulfurique dans des solvants organiques, caractérisé en ce qu'on effectue la sulfonation en présence de 0,01 à 0,9 mole d'halogénure d'hydrogène par mole d'anhydride sulfurique.

2. Procédé suivant la revendication 1, caractérisé en ce qu'on utilise le chlorure d'hydrogène comme halogénure d'hydrogène.

3. Procédé suivant les revendications 1 et 2, caractérisé en ce qu'on conduit la sulfonation à des températures de -40 à +20°C.

4. Procédé suivant les revendications 1 à 3, caractérisé en ce qu'on utilise le dichlorométhane comme solvant organique.